# EUROPEAN PATENT APPLICATION

(11) **EP 4 710 743 A1**
(43) Date of publication of application: **18.03.2026**
(21) Application number: 24200749.0
(22) Date of filing: 17.09.2024
(51) Int. Cl.: A01B 79/00, G06Q 50/02

(54) **SOIL PROPERTY INDICATOR SCORE AND SOIL HEALTH INDEX**

(71) Applicant: Yara International ASA, 0277 Oslo (NO)
(72) Inventor: BARIK, Muhammad, Tampa, 33602 (US); ARAB, Mehrdad, 48249 Dülmen (DE); ETICHA, Dejene, 0277 OSLO (NO)
(74) Representative: AWA Sweden AB

(57) **Abstract**

The present invention relates to a computer-implemented method and system for determining a soil property indicator score, wherein the method comprises selecting at least one soil property; receiving soil property data for a plurality of locations within a region; determining an inherent site-specific factor; receiving geospatial data for the plurality of locations; and assigning a geospatial class to at least a part of the plurality of locations, based on the inherent site-specific factor and on the received soil property data; identifying land parcels within the region, each land parcel including two or more locations with the same geospatial class; receiving predetermined value data for said soil property for a specific location within the region; determining the geospatial class of the land parcel comprising the specific location; identifying a subset of land parcels, each land parcel in the subset having the same geospatial class as the land parcel comprising the specific location; determining a scoring function for said soil property based on the received geospatial data of the subset of land parcels; and determining the soil property indicator score of the specific location based on the determined scoring function. Further embodiments relate to the generation of a soil health index and the generation of a recommendation and/or implementation of an agricultural practice.

## Description

### TECHNICAL FIELD

The present disclosure relates to the field of soil sciences. In particular, the present disclosure relates to a computer implemented method and system for determining a soil property indicator score for a geographical area, as well as a soil health index and a recommendation system to improve the field productivity or state.

### BACKGROUND

In order to feed a growing global population and a corresponding growth in global livestock, there exists an increasing demand for agricultural crops. Simultaneously, the available global cropland surface area is in steady decline, due to a variety of factors, such as a changing climate, nature restoration and land repurposing. Consequently, there is a clear need to improve productivity for existing agricultural fields, in order to address the conflicting developments of increasing production demands and declining productive area.

An important driver for agricultural field productivity is the state of the soil in a geographical area, in particular regarding soil factors such as inherent soil properties, soil nutrient levels, mechanical soil stability, soil resilience to environmental changes, potential for sustaining agricultural production and/or a well-balanced eco system. Besides agricultural productivity, these factors may also be of importance for other areas, such as estimating soil carbon storage capacities for carbon trading or restoring ecosystems and rural lands.

Providing accurate information on the state of the soil in a given geographical area is important with respect to agricultural purposes, such as in short and long-term planning of crop management. Thereby agricultural productivity and efficiency of processes, such as fertilization, may be significantly increased. Furthermore, as mentioned hereinabove, the state of the soil may be important for other types of land management. Examples of the latter are, for instance, rural and urban land management, monitoring of food chain security, climate change mitigation planning, and landscape conservation. In each of these latter cases, information on the state of soil in a geographical area may be vital for both planning, regulating, and project-implementation purposes.

Soil characteristics vary greatly depending on geographic locations and their respective weather, precipitation levels and temperature. Moreover, due to the high heterogeneity of soils within a region it is extremely cumbersome to set up a process which evaluates the suitability of soil and their quality for agricultural purposes.

Therefore, an improved approach which can determine the specific soil characteristics which render a soil healthy, productive and profitable is required.

Furthermore, there is a clear need for an easily accessible and reliable index for the state of the soil in a geographical area. Preferably, such index should be straightforward to understand and to transfer between various operators and organizations. Readily available and scientifically sound soil data and historic weather data should be utilized to determine the index, thereby improving index quality. Furthermore, the index should preferably be determined in an automated manner, avoiding elaborate and time-consuming field sampling and/or data verification and comparison. Finally, the index should preferably be scalable according to the extent of the geographical area of interest, to cover areas spanning from regions to continents.

### SUMMARY

The present disclosure concerns computer-implemented method for determining a soil property indicator score for a specific location.

According to a first aspect of the present disclosure, this and other objectives are achieved by a computer-implemented method for determining a soil property indicator score, wherein the method comprises selecting at least one soil property; receiving soil property data for a plurality of locations within a region; determining an inherent site-specific factor; receiving geospatial data for the plurality of locations; and assigning a geospatial class to at least a part of the plurality of locations, based on the inherent site-specific factor and on the received soil property data; identifying land parcels within the region, each land parcel including two or more locations with the same geospatial class; receiving predetermined value data for said soil property for a specific location within the region; determining the geospatial class of the land parcel comprising the specific location; identifying a subset of land parcels, each land parcel in the subset having the same geospatial class as the land parcel comprising the specific location; determining a scoring function for said soil property based on the received geospatial data of the subset of land parcels; and determining the soil property indicator score of the specific location based on the determined scoring function.

Following this approach, a measurable and clear valuation of a specific soil property can be achieved.

According to a further aspect of the present disclosure, the method further comprises selecting a plurality of soil properties and receiving said soil properties for the plurality of locations within the region; receiving predetermined value data for each of said plurality of soil properties for the specific location; determining a scoring function for each of said plurality of soil properties based on the received geospatial data of the subset of land parcels and determining a soil property indicator score for each of said plurality of soil properties of the specific location based on the scoring function.

Following this approach, a plurality of soil properties can be evaluated and individually assessed.

According to a further aspect of the present disclosure, the method further comprises determining a soil health index based on the soil property indicator score.

Following this approach, a full picture of the soil quality and status, considering different soil property indicator scores can be achieved.

According to a further aspect of the present disclosure, determining the scoring function further comprises determining the distribution of the soil property data in the land parcel subsets and fitting the scoring function to the determined distribution.

Following this approach the soil property indicator score is fine tuned to land parcel subsets of similar properties and therefore its relevance increased.

According to a further aspect of the present disclosure, assigning a geospatial class further comprises defining the classes based on the response of the soil property data such that, when determining ranges or subsets of the geospatial class, the soil property data presents a predetermined response.

Following this approach, the processing of the geospatial classes for the different inherent site-specific factors and the subsequent binning is simplified.

According to a further aspect of the present disclosure, the method further comprises determining a plurality of inherent site-specific factors and assigning a plurality of geospatial classes; and wherein each land parcel in the subset of land parcels has the same geospatial classes as the land parcel comprising the specific location.

Following this approach, the computational load of the current approach is lowered by binning the specific subset of land parcels in reduced groups or highly relevant and similar land parcels.

According to a further aspect of the present disclosure, the method further comprises the geospatial data comprising at least one of soil data and climatic data, and the geospatial classes comprise at least one of a soil class and a climatic class, wherein the soil data comprises soil texture data and wherein the climatic data comprises historic precipitation data and / or historic temperature data.

Following this approach, highly relevant classes for common soil properties are selected.

According to a further aspect of the present disclosure, the method further comprises at least one of the following: the soil class comprising a fine soil class, a medium soil class, or a coarse soil class; the climate class comprising a precipitation class and the precipitation class comprising a dry precipitation class, a moist precipitation class, or a wet precipitation class; the climate class comprising a temperature class and the temperature class comprising a cold temperature class, a temperate temperature class, a warm temperature class, or a hot temperature class.

Following this approach, most relevant classes and a specifically reduced subset of them is used for the selection of land parcel subsets.

According to a further aspect of the present disclosure, the method further comprises said soil property comprising at least one of: soil organic carbon content, plant available water capacity, nitrogen concentration, phosphorus concentrations, potassium concentration, pH value, or microbial biomass content.

Following this approach, the most relevant soil properties for the determination of soil quality are selected.

According to a further aspect of the present disclosure, the method further comprises the scoring function comprising a cumulative distribution function of said soil property and / or a threshold function for said soil property.

According to a further aspect of the present disclosure, the method further comprises at least one of determining the scoring function for plant available water capacity based on a cumulative distribution function of the soil class of the land parcels in the subset; determining the scoring function for the soil organic carbon content, nitrogen concentration, and / or microbial biomass content, based on a cumulative distribution function of the at least one soil class and climatic class and determining the scoring function for phosphorus, potassium and pH is based on a threshold function based on the at least one of a soil class and climatic class.

Following this approach, an improved evaluation of the relevant soil properties is achieved.

According to a further aspect of the present disclosure, the method further comprises determining an agricultural practice based on the soil property indicator score.

Following this approach, concrete actions which improve the actual state of the soil can be carried out.

According to a further aspect of the present disclosure, the method further comprises generating instructions for an agricultural system or vehicle to carry out the agricultural practice.

Following this approach, an automatic or semi-automatic process can be initiated to carry out the determined agricultural practice.

According to further aspects, a system, a data processing apparatus, a computer-readable storage medium and a computer program product configured to carry out the above discussed methods are envisaged within the present disclosure.

### BRIEF DESCRIPTION OF THE FIGURES

The following description of the figures of specific embodiments of the disclosure is only given by way of example and is not intended to limit the present disclosure, its application or use. In the figures, identical reference numerals refer to the same or similar parts and features.
Fig. 1A shows a schematic embodiment of a system according to an embodiment of the present disclosure.
Fig. 1B shows a representation of an agroforestry area according to one field of application of the present disclosure.
Fig. 2A shows a representation of soil parcels with soil classes according to an embodiment of the present disclosure.
Fig. 2B shows a representation of soil parcels with temperature classes according to an embodiment of the present disclosure.
Fig. 2C shows a representation of soil parcels with precipitation classes according to an embodiment of the present disclosure.
Fig. 3 shows a distribution of the different bins for a region according to an embodiment of the present disclosure.
Fig. 4 shows a diagram flow of a method according to an embodiment of the present disclosure.
Fig. 5 shows a diagram flow of a method according to an embodiment of the present disclosure.
Fig. 6A and 6B show different scoring functions according to different embodiments of the present disclosure.
Fig. 7A shows a triangle diagram of USDA soil texture classes as a function of particle size fractions, as used in the present disclosure.
Fig. 7B shows schematic precipitation classes for a specific embodiment in a predefined geographical area.
Fig. 7C shows schematic temperature classes for a specific embodiment in a predefined geographical area.
Figure 8 show soil property indicator scores and a soil health index for three locations according to an embodiment of the present disclosure.

### DETAILED DESCRIPTION

Unless otherwise defined, all terms used in disclosing the application, including technical and scientific terms, have the meaning as commonly understood by one of ordinary skill in the art to which this application belongs. By means of further guidance, term definitions are included to better appreciate the teaching of the present application.

In the following passages, different aspects of the application are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "an embodiment" means that a particular feature, structure or characteristic described in connection with the embodiment is included in at least one embodiment of the present application. Thus, appearances of the phrases "in one embodiment" or "in an embodiment" in various places throughout this specification are not necessarily all referring to the same embodiment but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the application, and form different embodiments, as would be understood by those in the art. For example, in the appended claims, any of the claimed embodiments can be used in any combination.

A computer-implemented method according to the present disclosure is next described. According to the method a soil property indicator score for at least one soil property is determined for a geographical area. According to a further embodiment, the computer-implemented method may determine a soil health index. The soil health index may provide an indication of the quality and suitability of the soil for agricultural purposes and/or the need for carrying out agricultural measures, such as irrigation, fertilization or other mechanical or chemical measure to address specific issues. Alternatively, the soil health index may represent a soil degradation index.

According to the present disclosure, an indicator score is a value, whether numerical or alphanumerical, rating a specific property. This rating is used to standardize values in different scales and ranges in a way that an untrained eye can understand the actual qualitative value of a measurement or soil property. Since soil properties are region dependent and inter-related, aiming to understand single (or multiple) values of a specific soil property is cumbersome and methods for the application of machine learning approaches ingesting the massive soil data libraries have been attempted, resulting in computationally expensive and intensive approaches. Hence, the soil property indicator score of the current disclosure aims at achieving a comprehensive and reliable indicator score which improves the computational effort needed for its determination.

The geographical area may comprise a region within a state, such as the Mid-West in the USA, or the Po delta in Italy. Alternatively, the geographical region may comprise one or more countries, such as the state of Germany and/or France, continental Europe or any other suitable region. A map of the geographical area may preferably be provided. Within the geographical area, a plurality of locations with soil property data and geospatial data is provided. The coordinates of each location in the geographical area and the map of the geographical area may, for instance, be stored in the memory of a computing device executing the method of the present disclosure, or in an external database/server. As detailed below, the geographical area may comprise a plurality of land parcels. A land parcel comprises a sub-area of the geographical area, such as an agricultural land delimited by a specific boundary, or a cluster of agricultural lands including, at least partially, non-agricultural elements like forests, rivers, roads and/or urban elements.

According to the method of the present disclosure, at least one soil property, for which the soil property indicator score will be determined, is selected. Based on the soil property selected, data of said soil property for a plurality of locations within a specific region is received. That is, if a soil property indicator score is intended for soil pH, the method of the current disclosure would receive from available stored soil data or soil databases soil pH data for a plurality of locations.

Further, based on the selected at least one soil property, at least one inherent site-specific factor is determined. According to the current disclosure, an inherent site-specific factor may comprise geospatial properties, i.e. information that describes events or other features with a location on or near the surface of the earth comprised in the regions within the geographical area used in the current method. Geospatial properties may comprise at least one of a soil property and climatic date, like soil data and/or weather (average precipitation, average temperature, maximum daily temperature, minimum daily temperature, sun irradiance, average wind speeds and direction and so on). Determining an appropriate inherent site-specific factor is essential for the current approach. Based on the selected at least one soil property for which the indicator score shall be determined, at least one inherent site-specific factor which is correlated with the distribution of the soil property over the geographical area is determined.

According to the method of the present disclosure, to each of a plurality of locations in the geographical region, a geospatial class is assigned, as will be detailed below. According to current method, a geospatial class is generated based on the determined inherent site-specific factor and on the received soil property data. For example, based on a predetermined soil property, like plant available water capacity, soil texture may be selected as inherent site-specific factor and classes for the soil texture may be generated and assigned to at least a part of the plurality of locations, as explained below.

Further, according to the current method, land parcels within the region with the same geospatial class are identified, wherein the land parcels comprise at least two locations with the same geospatial class and predetermined value data for said soil property for a specific location within the determined region is received.

According to the current disclosure, said predetermined value data for said soil property may comprise lab results of a specific soil property carried out by a farmer or an agronomist, but may be as well determined by ground sensors or remote monitoring methods. Soil property value data may be received by means of input unit, wherein a user, farmer or agronomist intending to evaluate at least one soil property of a soil sample might input the soil property value, but may be provided by usual communication networks from database or other equivalent methods. It is at this moment that the advantages of the current disclosure become clearer since said soil predetermined value data needs processing and evaluation in order to assess the quality of the soil for which said soil predetermined value data has been obtained. Despite the knowledge of the farmers or agronomists or other companies' representatives, it is difficult to evaluate a specific parameter for a soil within a specific region per se, since many different factors are involved for determining the overall quality of soil. Selecting the relevant factors and finding comparable land parcels which are representative for said determination is crucial and addressed by the current disclosure.

According to the current disclosure, said soil property value data may comprise any property representing the properties of a soil, such as physical, chemical, biological properties. According to the present disclosure, the property may be at least one of soil organic carbon (SOC) content, plant available water capacity, nutrient concentration (N, P, K, Mg, ...), pH value, soil particle aggregation, bulk density, porosity, plasticity, consistence, microbial mass content but not limited to these specific values. When the property value data is not in a numerical format, different procedures to normalize, standardize and convert to a numerical scala to be analyzed and treated by a numerical method within the customary practice can be carried out.

According to the current method, the geospatial class of the land parcel comprising the specific location is determined, which further allows identifying a subset of land parcels, each land parcel in the subset having the same geospatial class as the land parcel comprising the specific location, upon which a scoring function for said soil property is determined based on the received geospatial data of the subset of land parcels and a respective soil property indicator score can be determined, based on the determined scoring function. This will be further elaborated below with reference to the specific figures and specific embodiments.

Following this approach, relevant land parcels, with common properties to the specific location, are determined, allowing this the determination of representative scoring functions allowing the rating of the predetermined value data with a comparable set of soil properties.

Further advantageous embodiments will be detailed below, which will address specific improvements of the current disclosure and will be made clear with the following passages describing the accompanying figures.

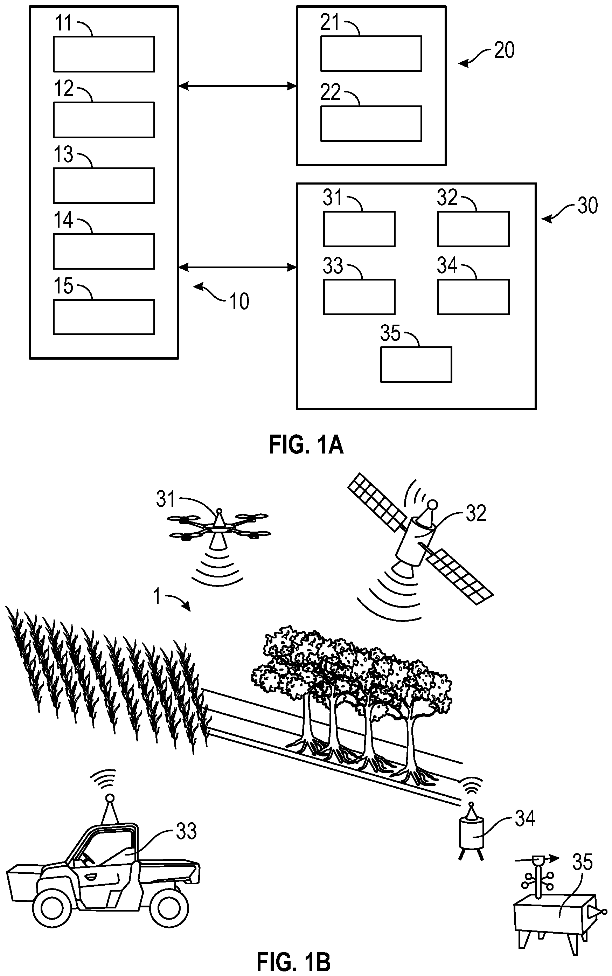

Fig. 1A shows a schematic representation of a system according to an embodiment of the present disclosure. System 10 represents a general purpose computer or any other kind of electronic processing device. System 10 may be a laptop computer but it is not limited to it and may be represented by a mobile device, a server - client architecture, or the like. System 10 comprises as usual a processing unit 11, a memory unit 12, an input unit 13 (e.g. a mouse, a keyboard a touch sensitive surface or similar), an output unit 14 (e.g. a display, a printer or the like) and accessory subsystems 15 (e.g. further processing units, gps unit, ...). System 10 may comprise further subsystems or units comprised within the usual elements of an electronic device and will not be further described herein. Correspondingly, each unit of system 10 is operatively connected with the remaining units as it is customary in the current practice and won't be further discussed here. System 10 is operatively connected to a plurality of data inputs represented by data input unit 20. Data input unit comprises at least one (21,22,...) database relating to soil data, weather data or other agronomical data. Public soil repositories are widely available (LUCAS, SSURGO, ...) and the present disclosure is not intended to be limited to any specific databases and the use of local databases or storage units comprising soil data is as well comprised within the teachings of the current disclosure. Weather data is as well publicly and widely available in form of different providers and may be as well obtained and/or simulated locally. Data input unit 20 may therefore be configured to retrieve and input data to system 10 of at least one of soil data, weather data or further agronomic data comprising, e.g., agricultural practices or products applied, past or current crop type and other crop data (sowing date, seed density, past yield, currently expected yield) present in the agricultural field of the specific location or region. Soil data may comprise at least one of soil organic carbon (SOC), plant Available Water Capacity (AWC), Nitrogen (N), Phosphorus (P), Potassium (K), pH, microbial biomass (MicBio), soil structure, bulk density, porosity, hydraulic conductivity, electric conductivity (EC), soil reflectance, cation exchange capacity (CEC) sodium adsorption ratio (SAR), soil respiration rate, but is not limited to these. Soil data may further comprise data related to other nutrients or chemical, physical or biological properties. Weather data may comprise at least one of winds, temperature, sun irradiance, sun hours, snow or frosting indicator scores, and the like. Precipitation data may comprise at least one of an amount of rain per square surface unit, average precipitation amount per time unit (day, week, season, year) or the like, while temperature data may comprise at least one of an average daily, weekly, monthly or yearly temperature, minimum and maximum temperatures and the like.

Fig. 1B shows a representation of an agroforestry area 1. Agroforestry areas usually comprise at least one of a crop, a land parcel and/or forests for agricultural or commercial uses. In such an area, different agricultural systems 30 may be present which, according to different embodiments of the present disclosure, are configured to be connected to system 10 by means of usual wired or wireless networks. For example, different devices like drones or other unmanned vehicles 31, tractors or other agricultural system or vehicles 33 may be present on site. These devices may be used for inspection, or to carry out agricultural practices which improve the state of the soil or its productivity, or may as well be configured to carry out measurements on the fields comprised in the agroforestry area. Within the present disclosure, other sensing platforms are considered which can be used, comprising amongst them remote or satellite sensing platforms 32, agricultural sensors 34 and/or weather stations 35.

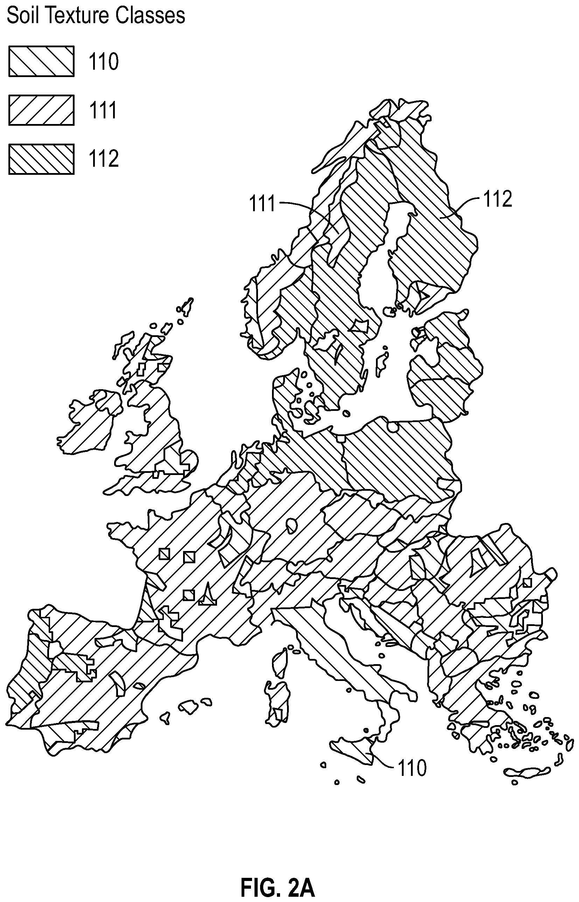

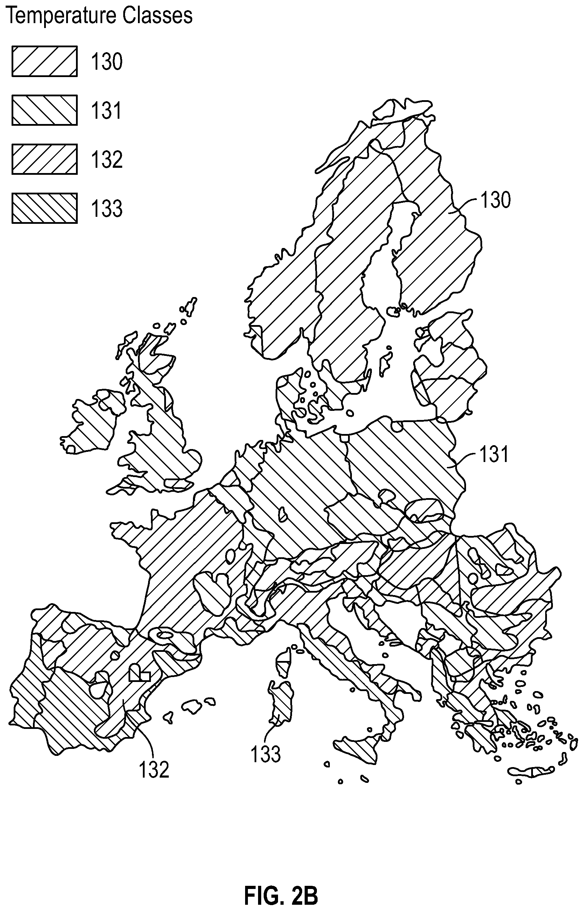

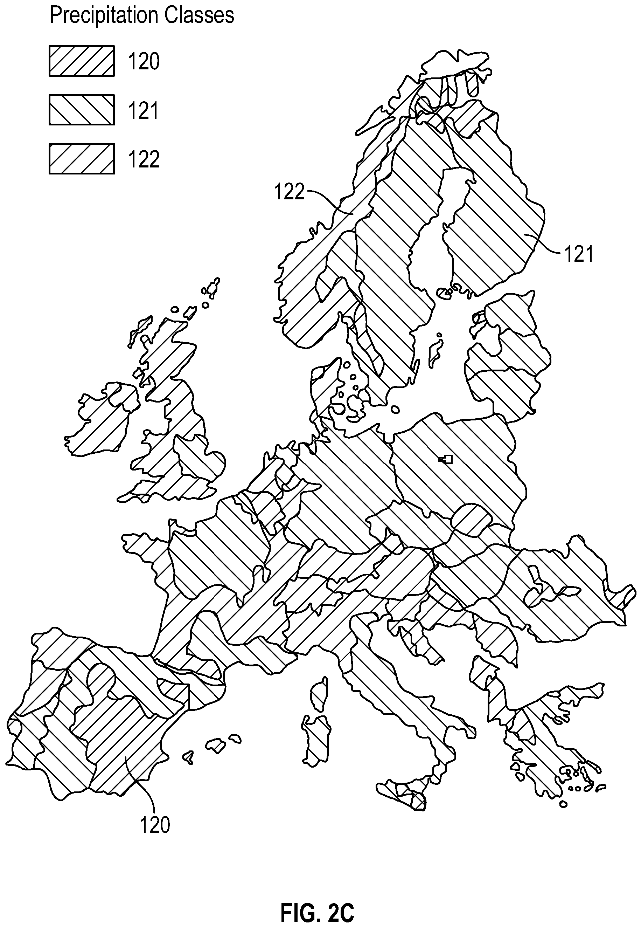

Figures 2A, 2B and 2C show different geospatial classes as used within the teachings of the different embodiments of the present disclosure.

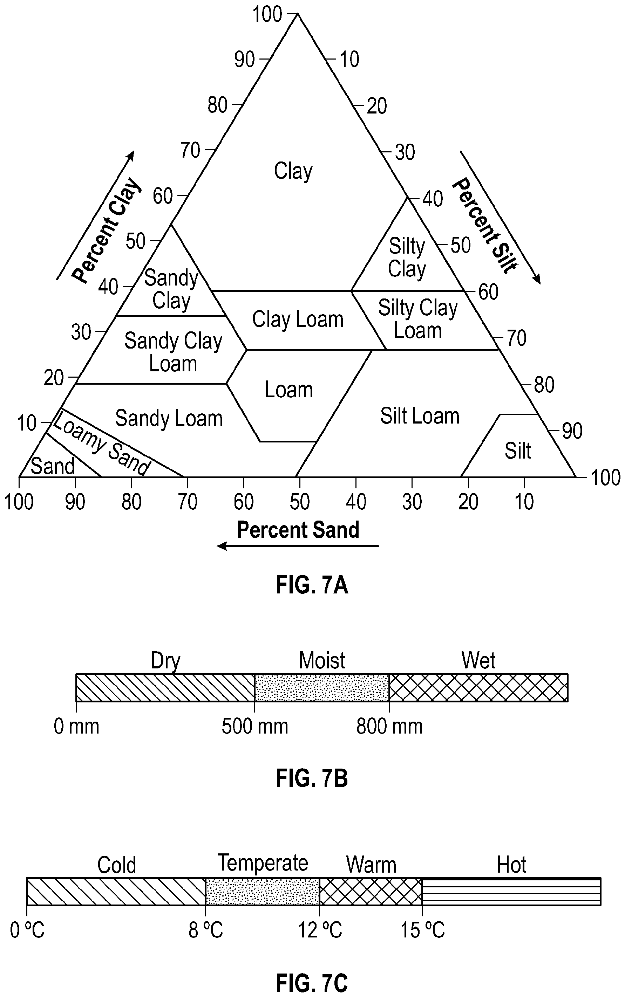

According to a further embodiment, the geospatial class comprises a soil class and the geospatial class data comprises soil texture data. Figure 2A shows such an embodiment where the geospatial class is based on soil texture and three classes are used for a specific geographic location. The regions comprising specific geographic locations are to be seen in Figure 2A with the different patterns and the reference signs 110, 111 and 112. Soil texture refers to the relative proportions of the three particle size fractions sand, silt and clay and reference will be made to Figure 7A as the usual established USDA soil texture classification. The sand, silt and clay particles in a soil are usually of mineral origin. As schematically shown in Figure 7A, twelve USDA soil texture classes can be defined as a function of the three soil particle size fractions. These USDA soil texture classes were first proposed by the United States Department of Agriculture (USDA) in 1938 and used nowadays by, amongst others, the USDA and the Food and Agriculture Organization (FAO). The soil particles in each of the three fractions have diameters less than 2 mm. Specifically, sand has a particle diameter from 0.05 mm to 2.0 mm, silt has a particle diameter from 0.002 mm to 0.05 mm, and clay has a particle diameter less than 0.002 mm. Clay particles exhibit colloidal properties and have a negative charge. Clay particles may normally have a flat and / or plate-like shape.

Soil texture may serve as a general indicator score of soil-related characteristics, such as fluid infiltration capacity and available water capacity, permeability, degree of aeration, drainage, and other physical soil characteristics.

With reference to Figure 7A, the twelve USDA soil texture classes as a function of the three soil particle size fractions are: I - sand, II - loamy sand, III - sandy loam, IV - sandy clay loam, V - loam, VI - silt loam, VII - silt, VIII - silty clay loam, IX - clay, X - clay loam, XI - sandy clay and XII - silty clay. Loam indicates a soil with predominantly sand and / or silt, and less clay.

In order to reduce the computational effort, the twelve USDA soil texture classes may be reduced to three soil classes. These three soil classes may comprise a coarse soil class, a medium soil class and a fine soil class. The coarse soil class may comprise the USDA soil texture classes I - sand, II - loamy sand, and III - sandy loam and are represented in Figure 2A by reference sign 112. The medium soil class may comprise the USDA soil texture classes IV - sandy clay loam, V - loam, VI - silt loam, and VII - silt and are represented in Figure 2A by reference sign 111. Finally, the fine soil class may comprise the USDA soil texture classes VIII - silty clay loam, IX - clay, X - clay loam, XI - sandy clay and XII - silty clay, and are represented in Figure 2A by reference sign 110. Alternatively, more than three soil classes may be used, such that each soil class includes fewer USDA soil texture classes. In some cases, up to twelve soil classes may be used, such that each soil class corresponds to one USDA soil texture class only. This may be advantageous when considering a limited geographical area, such as a region within a state, where soil variations may be limited.

According to the method of the present disclosure, a soil class is assigned to each of a plurality of locations in a geographical area, based on received soil texture data. Soil texture data may comprise measured and / or interpolated percentages of sand, silt and clay. Alternatively, soil texture data may comprise measured and / or interpolated soil particle diameter distributions. Measured data may be sampled by utilizing laser diffraction, sieving, hydrometer analysis, x-ray sedimention and / or expert-based analysis. Interpolated data may be obtained by spatial interpolation of data from locations with known soil textures. Spatial interpolation techniques may comprise nearest-neighbor interpolation, regular or irregular triangulated network interpolation, kriging, spline-based interpolation or other suitable multivariate interpolation.

Upon receiving soil texture data for a location, a soil class may be assigned. Assigning a soil class may be performed by comparing the measured and / or interpolated percentages of sand, silt and clay with the aforementioned USDA soil texture classes I - XII. Based on the result of the comparison the correct soil class, i.e., coarse, medium or fine, can be assigned to the location. Alternatively, assigning the soil class may comprise binning measured soil particle diameter distribution data in the three soil size fractions sand, silt and clay, to derive percentages of sand, silt and clay. The percentages of sand, silt and clay may then be compared with the USDA soil texture classes and a correct soil class may be assigned to the location, as described before. The assigned soil class may, for instance, be stored in the memory of a computing device on which the method of the present disclosure is executed, together with a reference to the location. Alternatively, the assigned class may be stored in an external database.

Next, soil parcels may be identified within the geographical area. A soil map may thereby be created, where the soil parcels are plotted onto the geographical area, as depicted in Figure 2A. Preferably, the soil parcels cover the entire geographical area. Each soil parcel includes neighboring first locations with the same soil class. All locations within a given soil parcel therefore have the same soil class. Boundaries of each soil parcel may, for instance, be computed by clustering techniques, by defining polygons, or by other suitable topological techniques. The soil parcels and/or soil map, may be stored in memory unit 12 or in an external memory, such as an online database, or an external disk. Hence, when identifying a subset of land parcels within the geographic region as displayed in Figure 2A, land parcels in said geographical area having the same soil texture as the land parcel comprising the specific location are selected. In this specific case, may the specific location be comprised within an area defined by reference sign 111 (i.e. medium soil class), the subset of land parcels shall be contained within areas marked with the same striped pattern, i.e. belonging to areas marked as 111, i.e. having a medium soil class.

According to a further embodiment, the geospatial class comprises a climatic class and geospatial data comprises climatic data. According to a further embodiment, the climatic class comprises a temperature class and climatic data comprises temperature data. Figure 2B shows such an embodiment where the geospatial class is based on temperature and four temperature classes have been generated for the specific region as shown in Fig. 2A. According to an embodiment, the four temperature classes may be selected as displayed in Figure 7C.

According to the current embodiment, when the geospatial class is based on temperature, a temperature class is assigned to each of the plurality of locations in the geographical area, based on received historic temperature data. Historic temperature data may, for instance, be derived from historical meteorological records for the geographical area. The historic temperature data may span a period of at least one year, preferably at least five years, more preferably at least ten years. The historic temperature data may be received from an external source, such as an online meterological database, or an external disk comprising such a meteorological database. Alternatively, historic temperature data may be received from an internal memory of a computing device executing the method of the present disclosure.

One or more temperature classes may be defined, as a function of average annual temperature in the geographical area. Preferably, the following four classes are defined: 'cold', `temperate', 'warm', and 'hot', represented respectively in Figure 2B by reference signs 130, 131, 132 and 133. The number and/or boundaries of the temprature classes may, again, depend on the geographical region. For the geographical area of Europe, for instance, cold is defined as an average annual temperature from 0 °C to 8 °C. Temperate is defined as an average annual temperature from 8 °C to 12 °C. Warm is defined as an average annual temperature from 12 °C to 15 °C. Hot is defined as an average annual temperature at or above 15 °C. A schematic not-to-scale representation of these four temperature classes for the geographical area of Europe is shown in Figure 7C, where the average annual temperature increases from left to right.

For the geographical area of South-East Asia, on the other hand, these temperature values are nessecarily different, due to the higher average annual temperatures and lower temperature variability as compared to Europe. The average annual temperature for South-East Asia as a whole is around 27 °C. Optionally, more than four temperature classes may be defined. This may be advantageous when considering crops with a high temperature sensitivity, requiring more detailed temperature information to be considered.

The defined temperature classes may be stored in memory unit 12 or in an external memory, such as an online database, or an external disk

Upon receiving historic temperature data for a location, a temperature class may be assigned. Assigning a temperature class may be performed by comparing the historical temperature data with the defined temperature classes. Based on the results of the comparison the correct temperature class, such as cold, temperate, warm or hot, can be assigned to the location. The assigned temperature class may, for instance, be stored in memory unit 12 together with a reference to the location. Alternatively, the assigned temperature class may be stored in an external database.

Next, temperature parcels may be identified within the geographical area. A temperature map may thereby be created, where the temperature parcels are plotted onto the geographical area. Preferably, the temperature parcels cover the entire geographical area. Each temperature parcel includes neighboring locations with the same temperature class. That is, all locations within a given temperature parcel share the same temperature class. Boundaries of each temperature parcel may, for instance, be computed by clustering techniques, by defining polygons or by other suitable topological techniques.

According to a further embodiment, the geospatial class comprises a climatic class and geospatial data comprises precipitation data. According to a further embodiment, the climatic class comprises a precipitation class and climatic data comprises precipitation data. Figure 2C shows such an embodiment where the geospatial class is precipitation and three classes have been generated for the specific region. According to an embodiment, the three temperature classes may be selected as displayed in Figure 7B.

According to the current embodiment, when the geospatial class is based on precipitation, a precipitation class is assigned to each of the plurality of locations in the geographical area, based on received historic precipitation data. Historic precipitation data may, for instance, be derived from historical meteorological records for the geographical area. The historic precipitation data may span a period of at least one year, preferably at least five years, more preferably at least ten years, most preferably at least thirty years.

One or more precipitation classes may be defined, as a function of average annual precipitation in the geographical area. Preferably, at least three precipitation classes are defined. These three precipitation classes may comrise a dry class, a moist class and a wet class, respectively represented in Figure 2C by reference signs 120, 121 and 122. The exact boundaries between the precipitation classes may depend on the location of the geographical area on earth. For Europe, for instance, the dry class comprises an average annual precipitation from 0 mm to 500 mm. The moist class comprises an average annual precipitation from 500 mm to 800 mm. The wet class comprises an average annual precipitation at or above 800 mm. A schematic not-to-scale representation of these three precipitation classes for the geographical area of Europe is shown in Figure 7B. In Figure 7B, the average annual precipitation increases from left to right, where the dry class is indicated with slanted stripes, the moist class is indicated by dots, and the wet class is indicated with a grid.

For the geographical area of South-East Asia, on the other hand, the precipitation classes are necessarily different, due to the higher average rainfall in the geographical area of South-East Asia. Average annual precipitation for South-East Asia is around 2000 mm and corresponding precipitation classes may be generated to span sufficiently the differences between regions.

Optionally, more than three precipitation classes can be used. Using more than three precipation classes may be beneficial for geographical areas where a wider variation in annual rainfall exists, or when end users require more detailed information. The additional precipitation classes 'arid' and 'very wet' may for example be defined. For the geographical area of Europe, the arid precipitation class can be defined as an average annual precipitation of 0 mm to 250 mm. The dry precipitation class is then redefined as comprising an average annual precipitation of 250 mm to 500 mm. With continued refernce to Europe, the very wet precipitation class can be defined as an average annual precipitation above 1200 mm. The wet precipitation class is then redefined as comprising an average annual precipitation from 800 mm to 1200 mm.

Upon receiving historic precipitation data for a location, a precipitation class may be assigned. Assigning a precipitation class may be performed by comparing the historical precipitation data with the defined precipitation classes. Based on the result of the comparison the correct precipitation class, i.e., dry, moist or wet, can be assigned to the location. The assigned precipitation class may, for instance, be stored in memory unit 12. Alternatively, the assigned precipitation class may be stored in an external database. The precipitation class may preferably be stored together with a reference to the location.

Next, precipitation parcels may be identified within the geographical area. A precipitation map may thereby be created, where the precipitation parcels are plotted onto the geographical area. Preferably, the precipitation parcels cover the entire geographical area. Each precipitation parcel includes neighboring locations with the same precipitation class. That is, all locations within a given precipitation parcel share the same precipitation class. Boundaries of each precipitation parcel may, for instance, be computed by clustering techniques, by defining polygons or by other suitable topological techniques. The precipitation parcels and / or precipitation map, may be stored in an external memory, such as an online database, or an external disk. Alternatively, precipitation parcels and / or precipitation map may be stored in memory unit 12.

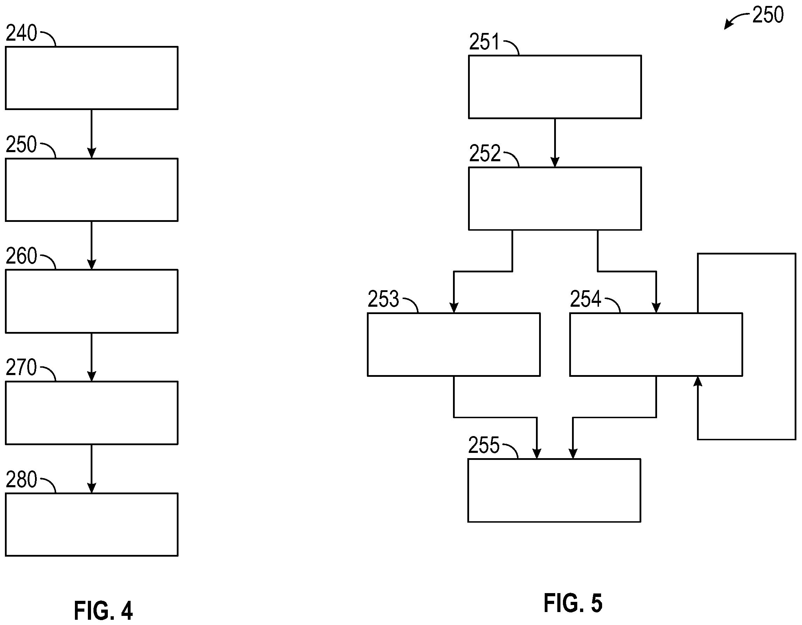

Soil texture data, temperature data and precipitation data may be preprocessed in order to remove outliers, normalize, and or standardize, according to usual practices and further details are not discussed here. Once the method of the current disclosure has ingested the required data, and after optional preprocessing, and the classes are generated, the method of the current disclosure will be explained following the diagram flows of Figure 4.

According to a further embodiment, the method further comprises determining a soil health index based on the soil property indicator score.

According to a further embodiment, the method further comprises selecting a plurality of soil properties and receiving said soil properties for the plurality of locations within the region, receiving predetermined value data for each of said plurality of soil properties for the specific location, determining a scoring function for each of said plurality of soil properties based on the received geospatial data of the subset of land parcels; and determining a soil property indicator score for each of said plurality of soil properties of the specific location based on the scoring function.

According to a further embodiment, the method further comprises determining a soil health index based on the plurality of soil property indicator scores.

Following this embodiment, a plurality of soil properties is analyzed in order to get a more detailed picture of the overall status of the soil. In this case, a plurality of inherent site-specific factors may be determined, and a plurality of geospatial classes may be assigned to at least a part of the plurality of locations, based on the plurality of inherent site-specific factors and on the received soil property data, as will be explained below.

Based on more comprehensive and detailed indicator scores of a plurality of soil properties, a soil health index may be generated or determined based on the specific indicator scores for the plurality of soil properties. The plurality of soil properties are processed 270 in order to determine 280 the soil health index. For example, according to a further embodiment, the soil health index may be determined 280 based on a weighted sum of the plurality of soil property indicator scores to obtain a soil health index indicative of the general soil health status. According to a further embodiment, the aggregated weighted sum may be scaled to suit a representative value (for example to a scale of 0 to 10, or 0 to 100) for easier communication and comparison. Both the soil property indicator score and the soil health index may be represented as a numerical value within a limited or unlimited range (e.g. in a scale from 0 to 100, or 0 to 10) or by a set of characters and signs (e.g. C, B, B-, B+, A, A-, A+, ...).

According to a further embodiment, assigning a geospatial class comprises defining the classes based on the response of the soil property data such that, when determining ranges or subsets of the geospatial class, the soil property data presents a predetermined response. As such, based on the definition of a specific class, e.g. a temperature class, and the corresponding ranges of each class (cold, mild, hot class...), the respective soil property data may present a linear, piecewise linear or quasilinear response, a quadratic response, a continuous or piecewise continuous response. Following this embodiment and the consideration of a plurality of site inherent specific factors and the respective classes, a suitable sorting into the defined bins can be achieved.

According to a further embodiment, the method of the current disclosure further comprises determining a plurality of inherent site-specific factors and assigning a plurality of geospatial classes;
and wherein each land parcel in the subset of land parcels has the same geospatial classes as the land parcel comprising the specific location. Based on a preliminary sensitivity test or equivalent analysis of the respective soil property data available for the at least one soil property to be analyzed, a single or a plurality of inherent site-specific factors can be determined. Based on a specific soil property (e.g. plant available water capacity), the inherent site-specific factor may be determined to be soil texture. This choice is inherent to the specific soil property. For example, for another soil property (e.g. soil organic carbon content), at least two inherent site-specific factors may be determined (e.g. soil texture and temperature). Based on the determined site-specific factor or factors, the respective geospatial classes and their number can be determined together with the specific respectively received soil property data related to the inherent site-specific factor.

According to a further embodiment, when a plurality of soil properties are evaluated, the method of the current disclosure may consider determining and assigning a plurality of geospatial classes, and hence, the subset of land parcels identified are based on having a unique combination of geospatial classes. This, while incrementing the complexity of the current method, will provide further advantages which will be made clear below.

According to this embodiment of the present disclosure, the unique combination of geospatial classes is defined as a bin. For the specific embodiment when three geospatial classes are to be selected, e.g., based on soil texture, precipitation and temperature, a bin is assigned 240 to each unique combination of soil class, temperature class and precipitation class. One such unique combination may, for example, be: 'dry precipitation class', `hot temperature class', and `coarse soil class'. For a geographical area with three soil classes, three precipitation classes and four temperature classes, such as for Europe as described hereinabove, there are thirty six unique combinations, or thirty six bins.

The bins may be stored in memory unit 12, or in an external memory, such as an online database, or an external disk.

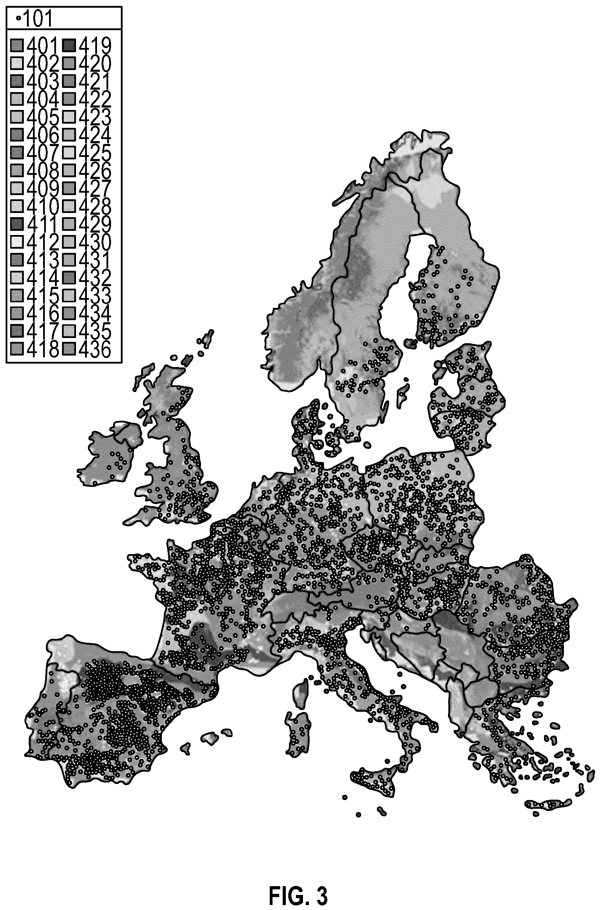

Next, according to the current embodiment, the soil parcels, precipitation parcels and temperature parcels are overlayed to identify bin parcels. The overlaying may comprise overlaying the soil map, the precipitation map and the temperature map, such that the external boundaries of the geographical area in all three maps match. The overlay of maps results in a bin map with bin parcels, where each bin parcel is associated with one bin. The bin parcels associated to any one bin may include at least ten, preferably at least twenty locations. The bin parcels and / or bin map, may be stored in an external memory, such as an online database, or an external disk. Alternatively, bin parcels and/or bin map may be stored in memory unit 12. A representation of the generated bins and the plurality of locations with a specific combination of geospatial classes being assigned, and hence defined as a bin according to the current specific embodiment of the present disclosure, is represented in Figure 3.

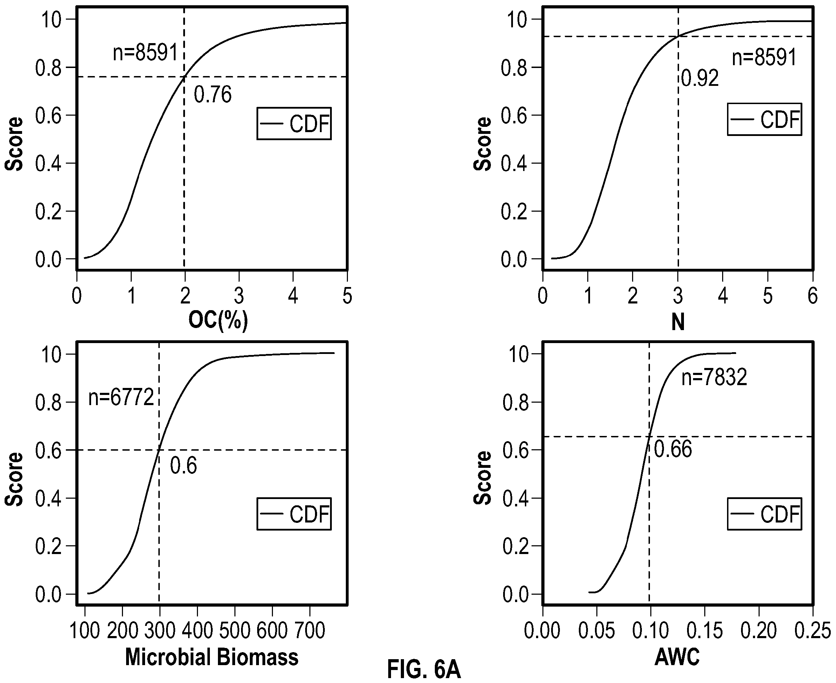

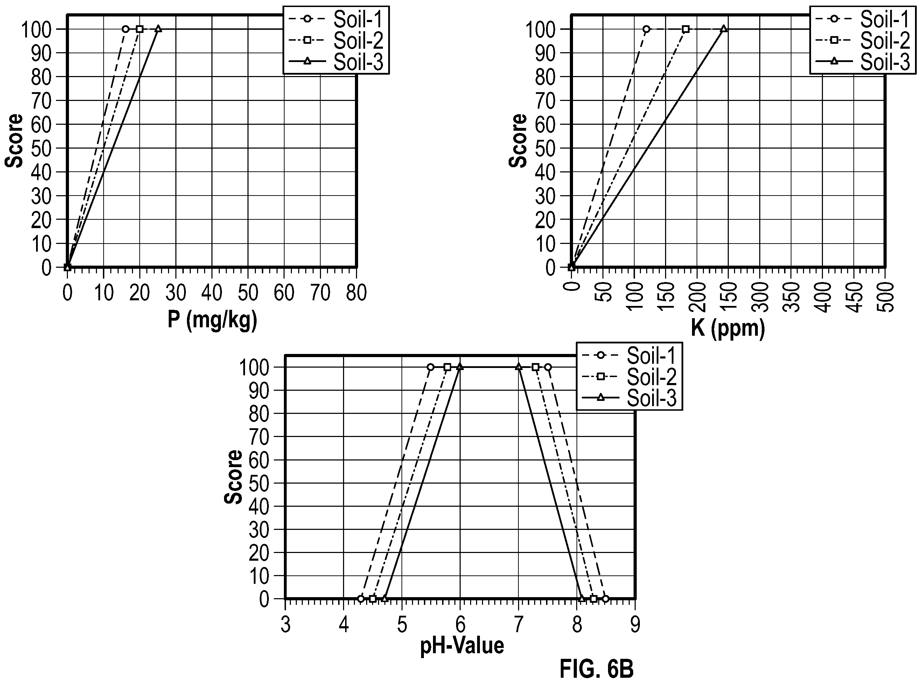

Determining 250 a scoring function is now described with reference to Figure 5 according to an embodiment of the present disclosure. A scoring function may comprise a mathematical function converting the value of the further soil property, at a location, into a relative dimensionless value between 0 and 1. Scoring functions along the teachings of the present disclosure are shown in Figures 6A and 6B.

Determining 250 a scoring function may comprise developing one or more scoring functions based on the number and type of soil properties to be analyzed. Once the number of scoring functions are selected, the method may further comprise defining 251 the scoring function curve type. By means of sensitivity analysis or exploratory analysis, regression or minization techniques, different types of scoring functions may be defined. Different types of scoring functions considered by the current disclosure comprise cumulative distribution functions, threshold functions, continuous probability functions, logarithmic, spherical, ranked probability or the like.

In a further embodiment of the present disclosure, selecting the scoring function type may comprise selecting 253 a cumulative distribution function (CDF), as represented in Figure 6A, which takes values between 0 and 1. In general, the cumulative distribution function Fx(x) of a real-valued variable X denotes the probability P(X ≤ x), that X takes a value less than or equal to x, or F_{X}(x) = P(X ≤ x). In general, F_{X}(x) is a non-decreasing function of X. The following values are provided for orientation with respect to specific locations and cannot be taken as a limitation.

The soil property `nitrogen content' is usually expressed in grams of nitrogen per kilogram of soil, or g/kg N. The cumulative distribution for nitrogen content can determined for each bin. The CDF increases from zero to unity, in the interval zero nitrogen content, or 0 g/kg N, to maximum nitrogen content, close to 6 g / kg N. Setting the value of x equal to x = 3.0 g/ kg N, the corresponding value of the CDF, or Fx(x = 3) can be established for each bin. For each bin this value of the CDF represents the score. For the geographical area of Europe, this yields a minimum score of 0.76 for the bin 'coarse', 'moist' and `temperate'. Likewise for the geographical area of Europe the maximum score is 1.0 for the bin 'fine', 'moist' and 'hot'.

Corresponding CDF's can be defined for other soil properties, such as the aforementioned soil organic carbon content, plant available water capacity, nutrient concentration, pH value, soil particle aggregation, bulk density and / or porosity, plasticity and / or consistence, soil color, or microbial mass content.

Soil organic carbon content (OC(%), in top left in Figure 6A), for example, is usually expressed in percentages by volume or by weight. The CDF increases from zero to unity, in the interval zero soil organic content to maximum organic carbon content, at about 5%. Setting the value of x equal to x = 2.0 %, the corresponding value of the CDF, or F_{X}(x = 2), can be established for each bin. For each bin this value of the CDF represents the score. For the geographical area Europe, this yields a minimum score of 0.26 for the bin 'coarse', 'moist' and 'cold'. Likewise for the geographical area of Europe the maximum score is 0.97 for the bin 'fine', 'dry' and `temperate'.

Microbional biomass, as another example, is usually expressed in microgram microbial biomass per gram of soil dry weight, or µg C_{mic} / g_{dry}. Analogously as described above for nitrogen content, taking the geographical region of Europe and setting x = 250 µg C_{mic} / g_{dry} , yields a minimum score of 0.01 for the bin 'coarse', 'wet' and 'temperate'. Likewise, the maximum score of 1.0 is achieved for bin 'fine', 'moist' and 'warm'.

In this manner scoring functions may be developed for each soil property separately. Individual soil properties may thus be rated separately, wherein the combination of soil texture and/or climate classes account for the spatial variability due to soil and climate conditions.

Advantageously, soil properties which may have different units and can normally not be compared directly or easily, can hereby be compared. Furhtermore, the scoring functions provide indications on possible margins of improvement for agricultural practice and may provide a farmer with important information to prioritize specific practices which can improve the soil of their agricultural fields. Furthermore, the rating may also provide indications to other stakeholders about the underlying properties of soil on a land parcel.

In a further embodiment of the present disclosure, selecting the scoring function type may comprise selecting 254 a threshold function, as depicted in Figure 6B for P, K and pH values. Threshold functions may be defined like a low-, high-, or band-pass filter-like functions, wherein values of the soil property, bigger, smaller or comprised outside a specific range, respectively, are rated with a lower score. For example, pH is only optimal when comprised in between a specific range of values, however variable, depending exclusively on the soil class. As such, as depicted in the lower image of Figure 6B, three different "band-pass" functions are defined for thre different classes of soil.

According to a further embodiment, once the scoring function type has been determined, determining the scoring function may further comprise determining the distribution of the soil property data in the land parcel subsets and fitting 255 the scoring function to the determined distribution.

Due to the binning procedure, creating different sets of land parcels for specific parcels with common factors, not all factors can be accounted for which have an influence in the soil properties. In order to adress non-homogeinities of specific soil properties a specific scoring function can be determined.

According to a further embodiment, the method of the current disclosure further comprises at least one of determining the scoring function for plant available water capacity based on a cumulative distribution function of the soil class of the land parcels in the subset; determining the scoring function for the soil organic carbon content, nitrogen concentration, and / or microbial biomass content, based on a cumulative distribution function of the at least one soil class and climatic class; or determining the scoring function for phosphorus, potassium and pH is based on a threshold function based on the at least one of a soil class and climatic class.

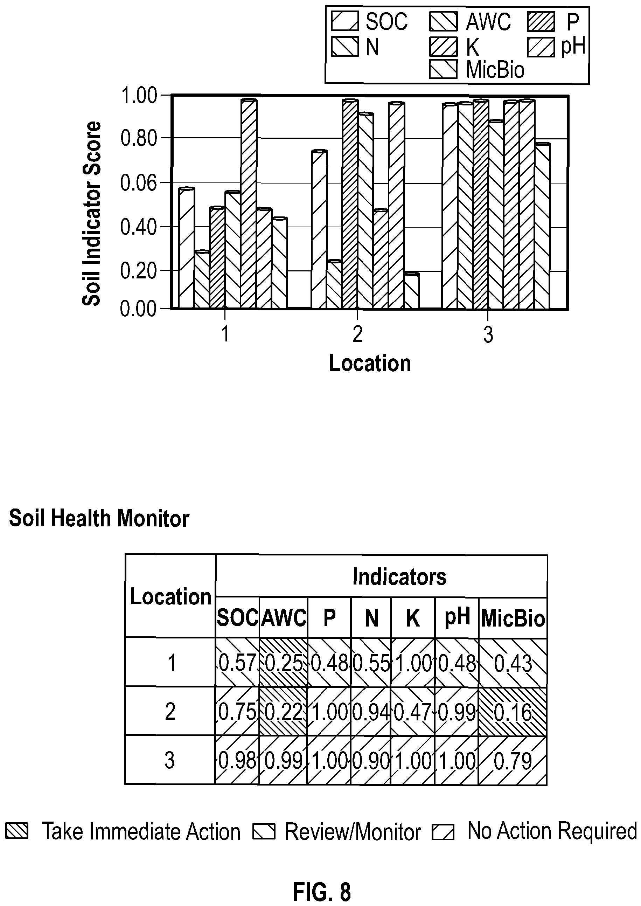

Following the different embodiments explained above, the advantages can be explained with respect to Figure 8. In the upper diagram of the figure, a soil property indicator score for a plurality of soil properties at a plurality of locations 1, 2 and 3 are represented and therefore an individual evaluation of the different soil property indicator scores can be carried out which takes into account the plurality of inherent site-specific factors and the soil properties at a plurality of locations other than the sampled locations 1, 2 and 3. As expressed above, the soil health indicator score may be referred to an individual soil property indicator score, or more preferably, to a plurality of soil property indicator scores. For example, analyzing the upper figure, it can be stablished that location 1 has an optimum Potassium (K) indicator score, but average indicator scores for the other soil properties analyzed and even critical conditions regarding the plant available water capacity (AWC). Location 2 presents an average Potassium (K) indicator score but better other indicator scores. Location 3 shows almost optimum values for the soil property indicator scores and therefore represents much healthier soils.

This can be as well assessed with respect to the lower diagram of Figure 8, wherein for each location an indicator score of the urgency of each agricultural practice which may be needed can be assessed. Location 1 is in need of immediate actions for improving the plant available water capacity or adapting the agricultural practice to such a condition. The remaining indicator scores point towards the need for surveillance, or the need for monitoring, and may as well be considered for generating an agricultural practice recommendation. Location 3 however, is at it best position regarding the analyzed indicator scores and no action is required.

According to a further embodiment, based on the at least one soil property indicator score, the method of the current disclosure may be configured to determine or adapt an agricultural practice based on the at least one soil property indicator score. For example, the method of the current disclosure may determine that the pH of the determined first location is too high and recommend the user to take appropriate action to improve the soil quality or adapt a fertilizer recommendation or application of other chemical products based on the determined pH indicator score. Further examples include, based on the available water content of the soil analyzed to improve an irrigation or fertigation plan which takes into consideration the plant available water content indicator score. Other actions may include the application of an agricultural product (fertilizer, pesticide, organic matter, biostimulants or inhibitors), irrigation, or soil handling practices based on the particular soil property indicator score determined.

According to a further embodiment, the method further comprises generating instructions for an agricultural system to carry out the agricultural practice.

Following the previous embodiment, with reference to Figure 1B, the current method may be further configured to communicate with agricultural system or vehicle 33 via standard networks and/or produce a machine-readable script which can be uploaded to the agricultural system or vehicle 33 for the agricultural practice to be carried out. The agricultural practice may then be carried out in an automatic or semi-automatic manner.

All references cited in the present specification are hereby incorporated by reference in their entirety. In particular, the teachings of all references herein specifically referred to are incorporated by reference.

While the present disclosure has been illustrated by a description of various embodiments and while these embodiments have been described in considerable detail, it is not the intention of the applicant to restrict or in any way limit the scope of the appended claims to such detail. Additional advantages and modifications will readily appear to those skilled in the art. The disclosure in its broader aspects is therefore not limited to the specific details, representative apparatus and method, and illustrative example shown and described.

In the present description of the application, reference is made to the accompanying drawings that form a part hereof, and in which are shown by way of illustration only of specific embodiments in which the application may be practiced. Parenthesized or emboldened reference numerals affixed to respective elements merely exemplify the elements by way of example, with which it is not intended to limit the respective elements. It is to be understood that other embodiments may be utilized and structural or logical changes may be made without departing from the scope of the present application. The following detailed description, therefore, is not to be taken in a limiting sense, and the scope of the present application is defined by the appended claims. The terms "comprises," "comprising,", "including," and "having," are inclusive and therefore specify the presence of stated features, integers, steps, operations, elements, or components, but do not preclude the presence or addition of one or more other features, integers, steps, operations, elements, components, or groups thereof. The method steps, processes, and operations described herein are not to be construed as necessarily requiring their performance in the particular order discussed or illustrated, unless specifically identified as an order of performance. It is also to be understood that additional or alternative steps may be employed.

The use of the expression "at least" or "at least one" suggests the use of one or more elements, as the use may be in one of the embodiments to achieve one or more of the desired objects or results.

The process steps, method steps, algorithms or the like may be described in a sequential order, such processes, methods and algorithms may be configured to work in alternate orders. In other words, any sequence or order of steps that may be described does not necessarily indicate a requirement that the steps be performed in that order. The steps of processes described herein may be performed in any order practical. Further, some steps may be performed simultaneously, in parallel, or concurrently. Various methods described herein may be practiced by combining one or more machine-readable storage media containing the code according to the present disclosure with appropriate standard computer hardware to execute the code contained therein. An apparatus for practicing various embodiments of the present disclosure may involve one or more computers (or one or more processors within a single computer) and storage systems containing or having network access to computer program(s) coded in accordance with various methods described herein, and the method steps of the disclosure could be accomplished by modules, routines, subroutines, or subparts of a computer program product. The scope of the disclosure is determined by the claims that follow. The disclosure is not limited to the described embodiments, versions or examples, which are included to enable a person having ordinary skill in the art to make and use the disclosure when combined with information and knowledge available to the person having ordinary skill in the art.

## Claims

1. A computer-implemented method for determining a soil property indicator score, the method comprising:
- selecting at least one soil property;
- receiving soil property data for a plurality of locations within a region;
- determining an inherent site-specific factor based on the at least one soil property;
- receiving geospatial data for the plurality of locations; and
- assigning a geospatial class to at least a part of the plurality of locations, based on the inherent site-specific factor and on the received soil property data;
the method further comprising:
- identifying land parcels within the region, each land parcel including two or more locations with the same geospatial class;
- receiving predetermined value data for said soil property for a specific location within the region;
- determining the geospatial class of the land parcel comprising the specific location;
- identifying a subset of land parcels, each land parcel in the subset having the same geospatial class as the land parcel comprising the specific location;
- determining a scoring function for said soil property based on the received geospatial data of the subset of land parcels; and
- determining the soil property indicator score of the specific location based on the determined scoring function.

2. The computer-implemented method of claim 1, wherein the method further comprises:
- selecting a plurality of soil properties and receiving said soil properties for the plurality of locations within the region;
- receiving predetermined value data for each of said plurality of soil properties for the specific location;
- determining a scoring function for each of said plurality of soil properties based on the received geospatial data of the subset of land parcels; and
- determining a soil property indicator score for each of said plurality of soil properties of the specific location based on the scoring function.

3. The computer-implemented method of claim 1 or 2, wherein the method further comprises determining a soil health index based on the soil property indicator score.

4. The computer-implemented method of any one of claims 1 to 3, wherein determining the scoring function comprises determining the distribution of the soil property data in the land parcel subsets and fitting the scoring function to the determined distribution.

5. The computer-implemented method of any one of claims 1 to 4, wherein assigning a geospatial class comprises defining the classes based on the response of the soil property data such that, when determining ranges or subsets of the geospatial class, the soil property data presents a predetermined response.

6. The computer-implemented method of any one of claims 1 to 5, further comprising
determining a plurality of inherent site-specific factors and assigning a plurality of geospatial classes;
and wherein each land parcel in the subset of land parcels has the same geospatial classes as the land parcel comprising the specific location.

7. The computer-implemented method of any one of claims 1 to 6, wherein the geospatial data comprises at least one of soil data and climatic data, and the geospatial classes comprise at least one of a soil class and a climatic class, wherein the soil data comprises soil texture data and wherein the climatic data comprises historic precipitation data and / or historic temperature data.

8. The computer-implemented method of claim 7, further comprising at least one of the following:
- wherein the soil class comprises a fine soil class, a medium soil class, or a coarse soil class;
- wherein the climate class comprises a precipitation class and wherein the precipitation class comprises a dry precipitation class, a moist precipitation class, or a wet precipitation class;
- wherein the climate class comprises a temperature class and wherein the temperature class comprises a cold temperature class, a temperate temperature class, a warm temperature class, or a hot temperature class.

9. The computer-implemented method of any one of claims 1 to 8, wherein said soil property comprises at least one of: soil organic carbon content, plant available water capacity, nitrogen concentration, phosphorus concentrations, potassium concentration, pH value, or microbial biomass content.

10. The computer-implemented method of any one of claims 1 to 9, wherein the scoring function comprises a cumulative distribution function of said soil property and / or a threshold function for said soil property.

11. The computer-implemented method of claim 10 when depending on 8, further comprising at least one of:
- determining the scoring function for plant available water capacity based on a cumulative distribution function of the soil class;
- determining the scoring function for the soil organic carbon content, nitrogen concentration, and / or microbial biomass content, based on a cumulative distribution function of the at least one soil class and climatic class;
- determining the scoring function for phosphorus, potassium and pH based on a threshold function based on the at least one of a soil class and climatic class.

12. The computer-implemented method of any one of claims 1-11, further comprising determining an agricultural practice based on the soil property indicator score.

13. The computer-implemented method of claim 12, further comprising generating instructions for an agricultural system or vehicle to carry out the agricultural practice.

14. A system configured to carry out the method according to any one of the claims 1-13.

15. A data processing apparatus comprising means for carrying out the method of any one of the claims 1-13.

16. A computer-readable storage medium comprising instructions which, when executed by a computer system, cause the computer system to carry out the method of any one of the claims 1 - 13.

17. A computer program product comprising instructions which, when the program is executed by a computer, cause the computer to carry out the method of any one of the claims 1-13.
